# EUROPÄISCHE PATENTSCHRIFT

(11) **EP 0 481 287 B1**
(45) Veröffentlichungstag und Bekanntmachung des Hinweises auf die Patenterteilung: **17.01.1996**
(21) Anmeldenummer: 91116809.4
(22) Anmeldetag: 02.10.1991
(51) Int. Cl.: C07C 39/17, C07C 37/20

(54) **Verfahren zur Herstellung von substituierten Cyclohexyliden-bisphenolen**
Method for the production of substituted cyclohexylidenebisphenols
Procédé pour la fabrication de cyclohexylidenebisphénols substitués

(30) Priorität: 13.10.1990 DE 4032595
(43) Veröffentlichungstag der Anmeldung: 22.04.1992
(73) Patentinhaber: BAYER AG, D-51368 Leverkusen (DE)
(72) Erfinder: Serini, Volker, Dr., W-4150 Krefeld (DE); Berg, Klaus D., Dr., W-4150 Krefeld (DE); Eitel, Alfred, Dr., W-4047 Dormagen (DE); Casser, Carl, Dr., W-5000 Köln 80 (DE); Waldmann, Helmut, Dr., W-5090 Leverkusen (DE); Hajek, Manfred, Dr., W-5090 Leverkusen (DE)

(56) Entgegenhaltungen:
- EP-A- 0 305 774
- EP-A- 0 359 953
- GB-A- 1 185 223
- US-A- 3 760 006

## Beschreibung

Die Erfindung betrifft ein Verfahren zur Herstellung von Cyclohexyliden-bisphenolen aus Phenolen in Gegenwart von Ionenaustauscher-Harzen und speziellen Cyclohexanonen.

Die erfindungsgemäß hergestellten Cyclohexyliden-bisphenole sind wertvolle Zwischenprodukte zur Herstellung von Polykondensationsprodukten. Insbesondere sind sie geeignet zur Herstellung von Polyetherketonen, Polyethersulfonen, aromatischen Polyestern, aromatischen Polyestercarbonaten und Polycarbonaten, die sich durch eine hohe Glastemperatur, besonders gutes Entformungsverhalten, außergewöhnlich gute Schmelzfließfähigkeit, insbesondere unter Berücksichtigung der hohen Glastemperatur und sehr gute UV-, Wärme- und Hydrolysestabilität auszeichnen.

Die Herstellung von Cyclohexyliden-Bisphenolen ist bekannt, z.B. aus den US-A 2,069,573, 2,894,004, 1,760,758, 2,538,725, 2,069,560, FR-A 1,559,848, GB-A 1 449 207, JP-A 1 268-640, J. Am. Chem. Soc. 61, 345 (1939). Sie werden in Gegenwart von Chlorwasserstoff oder Schwefelsäure als Kondensationskatalysator hergestellt. Als Cokatalysatoren können Schwefelverbindungen; z.B. n-Butylmercaptan hinzugegeben werden.

Ein Nachteil beim Arbeiten mit Chlorwasserstoff ist dessen hohe Korrosivität. Zur großtechnischen Herstellung ist daher eine korrosionsfeste Anlage erforderlich, die die Herstellungskosten der Bisphenole der Formel (I) deutlich erhöht. Die Korrosivität der HCl-haltigen Reaktionsgemische wird dadurch verstärkt, daß bei der Aufarbeitung noch Wasser zugesetzt wird. Die somit erhaltenen HCl-haltigen wäßrigen Lösungen sind schwierig aufarbeitbar. Werden die Reaktionslösungen bei der Aufarbeitung mit Basen, z.B. Natronlauge neutralisiert, entstehen zudem wäßrige, phenolhaltige Kochsalzlösungen.

Beim Arbeiten mit konzentrierter Schwefelsäure als sauren Kondensationskatalysator entsteht bei der Aufarbeitung durch Verdünnen mit Wasser verdünnte phenolhaltige Schwefelsäure.

Darüberhinaus müssen die meisten Verfahren zur Reinigung der Bisphenole der Formel (I), z.B. Abtrennung des nicht umgesetzten Phenols, bei Anwesenheit von Spuren löslicher Säure, im allgemeinen unterhalb 120°C im Hochvakuum durchgeführt werden.

Ein weiterer Nachteil bei der Verwendung von Chlorwasserstoff als sauren Kondensationskatalysator ist die geringe Ausbeute an Cyclohexylidenbisphenolen. So wird beispielsweise beim Einsatz von 3-Methylcyclohexanon nur eine 50%ige Ausbeute an Bisphenol der Formel (IV) erhalten (J. Am. chem. Soc. 61, 345 (1039)). Die Ausbeute an Bisphenol der Formel (IX) beträgt ebenfalls nur 46% (z.B. EP-A 368 604).

Für die Synthese von Bis-(4-hydroxyphenyl)alkanen durch Kondensation von Phenolen mit aliphatischen Carbonylverbindungen und zur Synthese von 1,1-Bis-(4-hydroxyphenyl)cyclohexan werden mit Vorteil wasserfreie, unlösliche Sulfonsäuregruppen enthaltende Kationenaustauscherharze eingesetzt (vgl. EP-A 342 758, EP-A 329 075, EP-A 319 327, DE-A 1 242 237, DE-A 2 811 182). Aus der GB-A 1185223 ist die Verwendung von Ionenaustauscherharzen, die mit einem Mercapto-Polymer modifiziert worden und, als Katalysator bekannt.

Die Nachteile, die bei der Verwendung eines flüssigen Katalysatorsystems auftreten, werden hierbei beseitigt.

Es wurde gefunden, daß sich speziell substituierte Cyclohexanone mit Phenolen in Gegenwart von stark sauren Kationenaustauschern zu den entsprechenden Bisphenolen in hohen Ausbeuten umsetzen lassen, wobei die Umsetzungsgeschwindigkeit durch Zugabe von schwefelhaltigen Cokatalysatoren, die den Reaktanten zugegeben oder chemisch an das Kationenaustauscherharz gebunden werden, erhöht werden kann.

Das erfindungsgemäße Verfahren ist überraschend, da der Einsatz von stark sauren Kationenaustauschern als Kondensationsmittel auf die Verwendung von Cyclohexanon und aliphatischen Carbonylverbindungen, insbesondere Aceton, beschränkt schien.

Gegenstand der Erfindung ist ein Verfahren zur Herstellung von substituierten Cyclohexylidenbisphenolen der Formel (I) 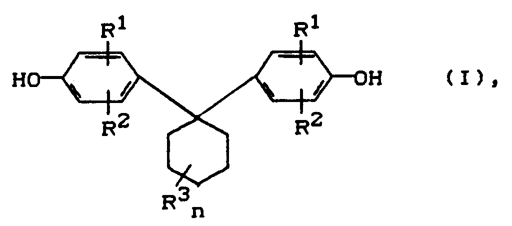
worin
- R¹ und R²: unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl und C₇-C₁₂-Aralkyl, bevorzugt Phenyl-C₁-C₄-Alkyl, insbesondere Benzyl,
- R³: einen C₁-C₁₂ Kohlenwasserstoffrest und
- n: die Zahl 1 und 2, mit der Maßgabe, daß geminale Substitution nicht umfaßt ist,
bedeutet,
durch Umsetzung von Phenolen der Formel (II) 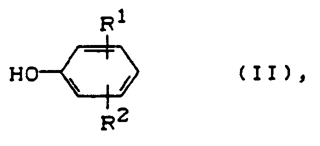
in welcher
- R¹ und R²: die bei Formel (I) angegebene Bedeutung haben,
in Gegenwart saurer, wasserfreier Kondensationskatalysatoren in Anwesenheit von organischen Schwefelverbindungen als Cokatalysator, mit Cyclohexanonen der Formel (III) 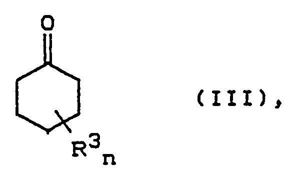
in welcher
- R³ und n: die bei Formel (I) angegebene Bedeutung haben,
eingesetzt werden, dadurch gekennzeichnet, daß als saure, wasserfreie Kondensationskatalysatoren, sulfonierte Styrol-Divinylbenzolharze, sulfonierte vernetzte Styrolpolymerisate, Phenol-Formaldehydsulfonsäureharze und 0,001 bis 0,2 Mol β-Mercaptopropionsäure oder 2 Mercaptoethylamin pro Mol Cyclohexanon der Formel (III) als Cokatalysator eingesetzt werden.

Beispielsweise können die Bisphenole der Formeln (IV) bis (X) hergestellt werden: 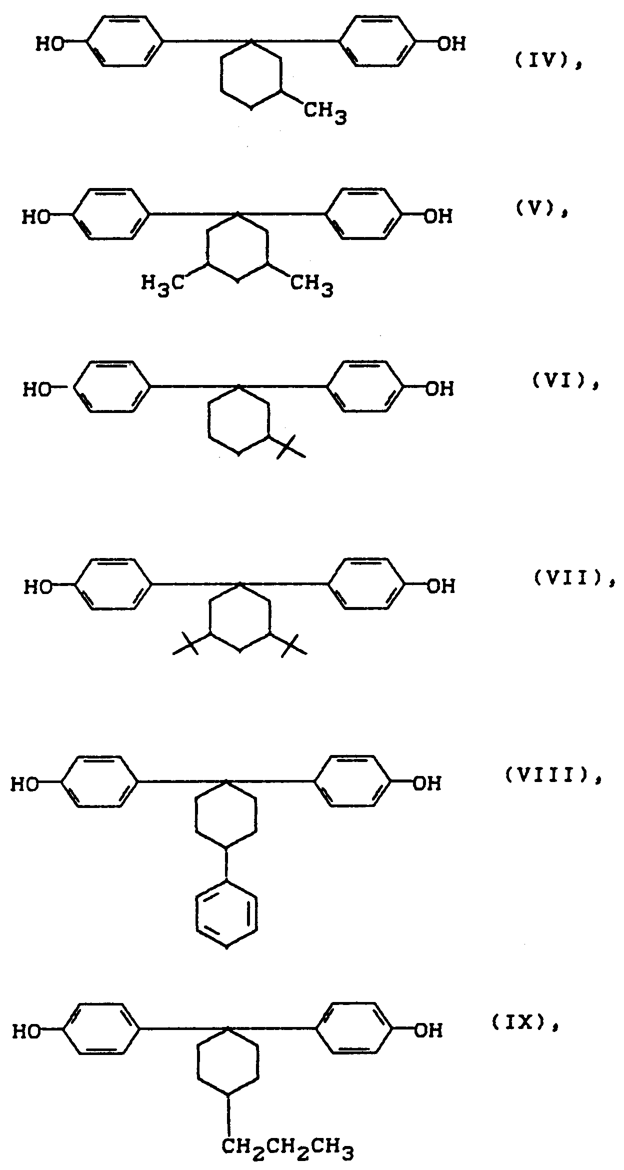 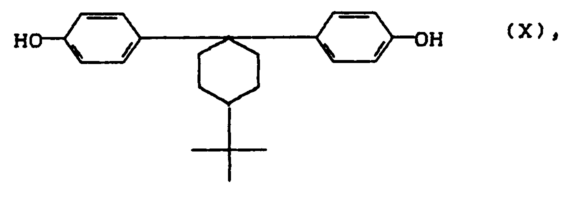
Geeignete Phenole der Formel (II) sind beispielsweise Phenol, 2,6-Dimethylphenol, 2-Chlorphenol, 2,6-Dichlorphenol, 2-Methylphenol, 2-Bromphenol, 2-tert.-Butylphenol, 2-Phenylphenol.

Geeignete Cyclohexanone der Formel (III) sind beispielsweise 3-Methylcyclohexanon, 4-Methylcyclohexanon, 3,5-Dimethylcyclohexanon, 3-tert.-Butylcyclohexanon, 3,5-Di-tert.-butylcyclohexanon, 3-Phenylcyclohexanon, 4-Phenylcyclohexanon, 4-Cyclohexylcyclohexanon, 4-tert-Butylcyclohexanon.

Bei dem Verfahren der Erfindung wird Phenol der Formel (II) im stöchiometrischen Überschuß zu Cyclohexanon der Formel (III) eingesetzt. Vorzugsweise werden 3 bis 30 Mol Phenol je Mol Keton eingesetzt. Ein Molverhältnis von Phenol zu Cyclohexanon von 6:1 bis 25:1 ist besonders bevorzugt.

Im erfindungsgemäßen Verfahren werden als saure Kondensationskatalysatoren sulfonierte Styrol-Divinylbenzolharze, sulfonierte vernetzte Styrolpolymerisate, Phenol-Formaldehydsulfonsäureharze verwendet. Diese sind unter dem Namen Amberlite®, DOWEX®, PermutitQH®, Chempro® und Lewatite® erhältlich. Polyperfluoralkylensulfonsäuren, erhältlich unter dem Namen Nafion®, können ebenfalls eingesetzt werden.

Die eingesetzten Ionenaustauscher haben eine H⁺-Ionenkapazität von 2 bis 7 mval/g Trockensubstanz und einen H₂O-Gehalt von 0,1 bis 2 Gew.-%.

Neben den sauren SO₃H-Gruppen können an den Ionenaustauschern entweder direkt an der Matrix oder über die Sulfonsäuregruppen noch Molekül-Gruppen mit SH-Funktion als Cokatalysator gebunden sein. Die Modifizierung der sauren Ionenaustauscher mit mercaptohaltigen Verbindungen ist bekannt (vgl. EP-A 305 774, EP-A 023 325, DE-A 2 722 683).

Der Mercaptogruppen-haltige Cokatalysator kann auch als niedermolekulare Verbindung in ungebundener Form z.B. als β-Mercaptopropionsäure, n-Alkylmercaptan, der Reaktionsmischung zugegeben werden. Hierbei wird der Cokatalysator in Mengen von 0.001-0.2 Mol pro Mol Cyclohexanon der Formel (II) eingesetzt.

Das erfindungsgemäße Verfahren kann kontinuierlich oder diskontinuierlich durchgeführt werden, wobei die kontinuierliche Fahrweise bevorzugt ist. Hierbei wird der saure Ionenaustauscher in einen geeigneten Reaktor, z.B. als Festbett ausgelegten vertikalen röhrenförmigen Reaktor, gefüllt und vom Reaktionsgemisch durchströmt. Die Fließgeschwindigkeit der Reaktionsteilnehmer wird so eingestellt, daß der gewünschte Cyclohexanonumsatz erreicht wird.

Beim diskontinuierlichen Verfahren ist es von Vorteil, die Reaktionsteilnehmer und den Katalysator gut mechanisch zu rühren, um in kurzen Zeiten hohe Ausbeuten zu erzielen.

Je nach Art des herzustellenden Cyclohexylidenbisphenols beträgt die Reaktionstemperatur 20 bis 150°C. Temperaturen von 35 bis 100°C sind bevorzugt. Bei der kontinuierlichen Fahrweise muß die Reaktionstemperatur so gewählt werden, daß das entstandene Bisphenol in der Reaktionsmischung im Austauscherbett nicht auskristallisiert.

Die Umsetzung kann auch in einem inerten Lösungsmittel durchgeführt werden. Geeignete Losungsmittel sind Toluol, Chlorbenzol, Methylenchlorid, Cyclohexan, Dichlorethan, Dioxan. Die Zugabe von inerten Lösungsmitteln ist dann von Vorteil, wenn das gebildete Cyclohexylidenbisphenol bei tiefen Temperaturen in Lösung gehalten werden soll. Die inerten Lösunsmittel können gegebenenfalls auch während der Umsetzung als Schleppmittel für das während der Reaktion gebildete Wasser dienen.

Die Isolierung der Cyclohexylidenbisphenole nach dem Verfahren der Erfindung kann in verschiedener Weise erfolgen. Beim diskontinuierlichen Verfahren kann z.B. die Reaktionsmischung bei einer Temperatur, bei der das Cyclohexylidenbisphenol in der Reaktionsmischung gelöst ist, durch Filtration vom unlöslichen Ionenaustauscherharz abgetrennt werden. Das Filtrat wird dann abgekühlt und/oder von überschüssigem Phenol befreit. Das hierbei anfallende Cyclohexylidenbisphenol-Phenol-Addukt wird filtriert und kann destillativ gereinigt werden, wobei das reine Cyclohexylidenbisphenol zurückbleibt und ein trockenes Phenoldestillat erhalten wird, das in das Reaktionsgemisch zurückgeführt werden kann. Die Spaltung des Cyclohexylidenbisphenol-Phenol-Adduktes kann auch mit Wasser und/oder einem Lösungsmittel erfolgen, in dem das Phenol löslich und das Bisphenol weitgehend unlöslich ist. Geeignete Lösungsmittel hierfür sind aliphatische und aromatische Kohlenwasserstoffe, beispielsweise Hexan, Cyclohexan, Toluol, Petrolether, Chlorbenzol.

Die Isolierung der Cyclohexylidenbisphenole kann auch in der Art erfolgen, daß man zur vom Ionenaustauscherharz abgetrennten Reaktionslösung ein Lösungsmittel zusetzt, wobei das Cyclohexylidenbisphenol oder das Cyclohexylidenbisphenol-Phenol-Addukt ausfällt und filtriert werden kann. Geeignete Lösungsmittel hierfür sind aliphatische und aromatische Kohlenwasserstoffe wie beispielsweise Hexan, Pentan, Petrolether, Toluol, Chlorbenzol.

Ist das Cyclohexylidenbisphenol in Gegenwart von sauren Ionenaustauscherharzen bei höheren Temperaturen instabil, so kann bei einer diskontinuierlichen Fahrweise die Aufarbeitung durch Zugabe eines Lösungsmittels erfolgen, das das Cyclohexylidenbisphenol und/oder sein Phenol-Addukt bei tiefen Temperaturen löst. Geeignete Lösungsmittel hierfür sind aliphatische und aromatische Ether, Ester und Alkohole wie beispielsweise Diisopropylether, Dioxan, Tetrahydrofuran, Essigsäuremethylester, Methanol, Ethanol, Isopropanol. Die Reaktionsprodukt-Lösung wird vom Ionenaustauscher abfiltriert und eingeengt. Zur Entfernung vom überschüssigen Phenol kann eine Extraktion mit Wasser bzw. eine Wasserdampfdestillation erfolgen. Ein weiteres effektives Verfahren zur Abtrennung von überschüssigem Phenol besteht darin, daß man die eingeengten Reaktionsprodukt-Lösungen mit einem Lösungsmittel versetzt, in dem das Phenol gelöst und das gewünschte Cyclohexylidenbisphenol weitgehend ungelöst ist. Die Isolierung des gewünschten Cyclohexylidenbisphenols aus der filtrierten Reaktionsproduktlösung kann auch über die Bildung des Cyclohexylidenbisphenol-Phenol-Adduktes wie oben beschrieben erfolgen.

Beim kontinuierlichen Verfahren erfolgt die Isolierung der gewünschten Cyclohexylidenbisphenole der Formel (I) analog der für das diskontinuierliche Verfahren dargestellten Arbeitsweise.

### Beispiele

### Beispiel 1

### 1,1-Bis-(4-hydroxyphenyl)-3-methylcyclohexan

Eine Mischung aus 2,4 Mol Phenol, 0,2 Mol 3-Methylcyclohexanon und 0,001 Mol β-Mercaptopropionsäure und 24 g trockenes Lewatit SC 102/H® wird bei 45°C unter N₂ gerührt. Nach 95%igem Umsatz des Ketons (GC-Kontrolle, Reaktionszeit ca. 10 h) werden 300 ml Methanol zugegeben und vom Ionenaustauscherharz filtriert. Der Ionenaustauscher wird dreimal mit je 100 ml Methanol gewaschen und im Vakuum bei 90-100°C getrocknet. Die vereinigten Methanollösungen werden im Vakuum eingeengt. Das hierbei anfallende Bisphenol-Phenol-Addukt wird filtriert und durch Extraktion mit Hexan vom überschüssigen Phenol befreit.

Ausbeute: 47 g ^ 83,6 % Fp. 172-173°C

### Beispiel 2

### 1,1-Bis-(4-hydroxyphenyl)-4-methylcyclohexan

Analog zu Beispiel 1 werden anstelle von 0.2 Mol 3-Methylcyclohexanon 0,2 Mol 4-Methylcyclohexanon eingesetzt.

Ausbeute: 48,5 g ^ 86 % Fp. 179-180°C

### Beispiel 3

### 1,1-Bis-(4-hydroxyphenyl)-4-tert.butylcyclohexan

Analog zu Beispiel 1 werden anstelle von 0,2 Mol 3-Methylcyclohexanon 0,2 Mol 4-tert.-Butylcyclohexanon eingesetzt.

Ausbeute: 55 g ^ 89 % Fp. 183°C

### Beispiele 4

### 1,1-Bis-(4-hydroxyphenyl)-3,5-ditert.-butylcyclohexan

Analog zu Beispiel 1 werden anstelle von 0,2 Mol 3-methylcyclohexanon 0,2 Mol 3,5-Di-tert.butylcyclohexanon eingesetzt.

Ausbeute: 62 g ^ 85 % Fp. 241-242°C

### Beispiel 5

Analog zu Beispiel 1 jedoch unter Verwendung des gebrauchten Ionenaustauscherharzes aus Beispiel 1

Ausbeute: 45 g ^ 84 % Fp. 171-172°C

### Beispiel 6

### 1,1-Bis-(4-hydroxyphenyl)-3-methylcyclohexan

Eine Mischung aus 6 Mol Phenol, 0.5 Mol 3-Methylcyclohexanon und 60 g trockenes Lewatit SC 102/H®, dessen saure Sulfonsäuregruppen zu 40% mit 2-Mercaptoethylamin neutralisiert wurden, wird bei 45-55°C gerührt. Nach 5 Stunden wird ein Umsatz von 97% erzielt. Das Reaktionsgemisch wird auf 65°C erwärmt und filtriert. Der Ionenaustauscher wird dreimal mit je 200 g Phenol bei 80°C gewaschen und filtriert, Aus den vereinigten Filtraten wird das Phenol durch Vakuumdestillation entfernt, der verbleibende Bisphenolrückstand wird aus Toluol umkristallisiert.

Ausbeute: 117,5 g ^ 86 % Fp. 171-172°C

### Beispiel 7

### 1,1-Bis-(4-hydroxyphenyl)-3-tert-butylcyclohexan

Analog zu Beispiel 6 werden anstelle von 0.5 Mol 3-Methylcyclohexanon 0.5 Mol 3-tert.butylcyclohexanon verwendet.

Ausbeute: 120 g ^ 76 % Fp. 194-195°C

### Beispiel 8

### 1,1-Bis-(4-hydroxyphenyl)-4-tert.-butylcyclohexan

Eine Mischung aus 24 Mol Phenol und 1 Mol 4-tert.-Butylcyclohexanon wurden durch eine vertikal angeordnete 1 m langen Glassäule mit einem Innendurchmesser von 8 cm im Aufstrom kontinuierlich gepumpt. Die Glassäule war mit 660 cm³ phenolfeuchtem Ionenaustauscherharz, dessen saure SO₃H-Gruppen zu 20% mit 2-Mercaptoethylamin neutralisiert waren, gefüllt. Als saures Kationenaustauscherharz wurde ein sulfoniertes Styroldivinylbenzol-Copolymerisat (Typ Lewatit SC 102/H®) mit 5 mval Säureaquivalenten pro Gramm Trockensubstanz verwendet, das durch Waschen mit Phenol vorgetrocknet und anschließend mit Toluol im Vakuum bis auf einen Wassergehalt von 0.5% getrocknet wurde (Trockengewicht 250 g). Die Säulentemperatur betrug 80-85°C. Bei einem Durchsatz von 180 g/h wurde ein Ketonumsatz von 98% erhalten. Die Selektivität an 1,1-Bis-(4-hydroxyphenyl)-4-tert.butylcyclohexan betrug 84%.

### Vergleichsbeispiele

### 1,1-Bis-(4-hydroxyphenyl)-3,3,5-trimethylcyclohexan

a) Eine Mischung aus 6 Mol Phenol, 0,5 Mol 3,3,5-Trimethylcyclohexanon und 60 g trockenem Lewatit SC 102/H® wird bei 40 bis 45°C unter N₂ gerührt. Nach 24 h betrug der Umsatz 8 %, nach 140 h 26 %.
b) Eine Mischung aus 6 Mol Phenol, 0,5 Mol 3,3,5-Trimethylcyclohexanon, 0,025 Mol β-Mercaptopropionsäure und 60 g trockenem Lewatit SC 102/H® wird bei 40 bis 45°C und N₂ gerührt. Nach 24 h betrug der Umsatz 26 %.
c) Analog zu b) werden anstelle von 0,025 Mol 0,06 Mol β-Mercaptopropionsäure eingesetzt. Nach 24 h betrug der Umsatz 55 %.

## Patentansprüche

1. Verfahren zur Herstellung von substituierten Cyclohexylidenbisphenolen der Formel (I) 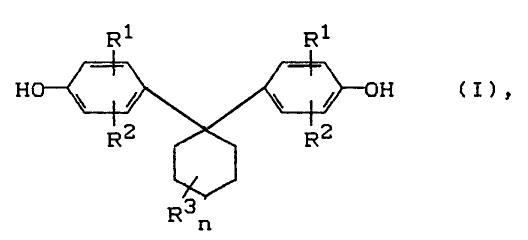 worin
R¹ und R² unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl und C₇-C₁₂-Aralkyl, bevorzugt Phenyl-C₁-C₄-Alkyl, insbesondere Benzyl,
R³ einen C₁-C₁₂ Kohlenwasserstoffrest und
n die Zahl 1 und 2, mit der Maßgabe, daß geminale Substitution nicht umfaßt ist,
bedeutet,
durch Umsetzung von Phenolen der Formel (II) 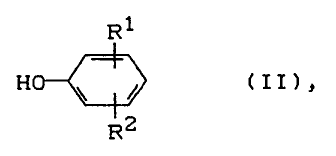 in welcher
R¹ und R² die bei Formel (I) angegebene Bedeutung haben,
in Gegenwart saurer, wasserfreier Kondensationskatalysatoren in Anwesenheit von organischen Schwefelverbindungen als Cokatalysator, mit Cyclohexanonen der Formel (III) 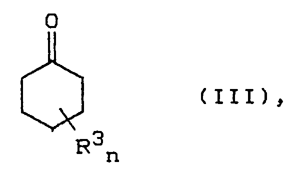 in welcher
R³ und n die bei Formel (I) angegebene Bedeutung haben,
eingesetzt werden, dadurch gekennzeichnet, daß als saure, wasserfreie Kondensationskatalysatoren, sulfonierte Styrol-Divinylbenzolharze, sulfonierte vernetzte Styrolpolymerisate, Phenol-Formaldehydsulfonsäureharze und 0,001 bis 0,2 Mol β-Mercaptopropionsäure oder 2 Mercaptoethylamin pro Mol Cyclohexanon der Formel (III) als Cokatalysator eingesetzt werden.

## Claims

1. Process for the production of substituted cyclohexylidene bisphenols of the formula (I) 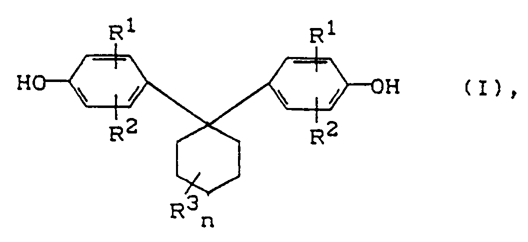 in which
R¹ and R² mutually independently mean hydrogen, halogen, preferably chlorine or bromine, C₁-C₈ alkyl, C₅-C₆ cycloalkyl, C₆-C₁₀ aryl, preferably phenyl and C₇-C₁₂ aralkyl, preferably phenyl C₁-C₄ alkyl, in particular benzyl,
R³ means a C₁-C₁₂ hydrocarbon residue and
n means the number 1 and 2, provided that geminal substitution is not included,
by reacting phenols of the formula (II) 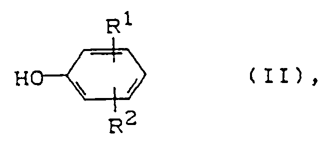 in which
R¹ and R² have the meaning stated for formula (I),
in the presence of acidic, anhydrous condensation catalysts in the presence of organic sulphur compounds as cocatalyst, with cyclohexanones of the formula (III) 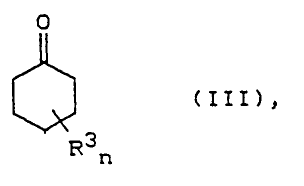 in which
R³ and n have the meaning stated for formula (I),
characterised in that sulphonated styrene-divinylbenzene resins, sulphonated crosslinked styrene polymers, phenol-formaldehyde sulphonic acid resins are used as the acidic, anhydrous condensation catalysts and 0.001 to 0.2 mol of β-mercaptopropionic acid or 2-mercaptoethylamine are used per mol of cyclohexanone of the formula (III) as the cocatalyst.

## Revendications

1. Procédé de préparation de cyclohexylidènebisphénols substitués de formule (I) : 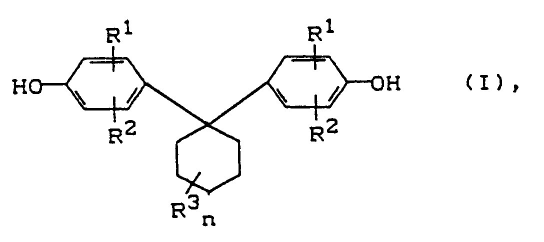 dans laquelle
R¹ et R² représentent chacun, indépendamment l'un de l'autre, l'hydrogène, un halogène, de préférence le chlore ou le brome, un groupe alkyle en C₁-C₈, cycloalkyle en C₅-C₆, aryle en C₆-C₁₀, de préférence phényle, ou aralkyle en C₇-C₁₂, de préférence phényl-alkyle en C₁-C₄, plus spécialement benzyle,
R³ représente un radical hydrocarboné en C₁-C₁₂ et
n est égal à 1 ou 2, sous réserve que les substitutions géminales sont exclues,
par réaction de phénols de formule (II) : 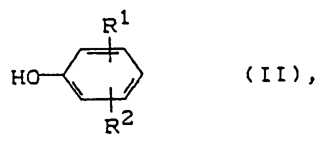 dans laquelle
R¹ et R² ont les significations indiquées en référence à la formule (I),
en présence de catalyseurs de condensation acides, anhydres, et en présence de dérivés organiques du soufre servant de catalyseurs auxiliaires, avec des cyclohexanones de formule (III) : 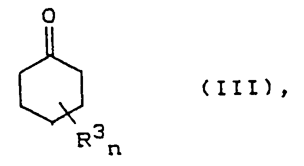 dans laquelle
R³ et n ont les significations indiquées en référence à la formule (I),
caractérisé en ce que l'on utilise en tant que catalyseurs de condensation acides, anhydres, des résines styrène-divinylbenzène sulfonées, des polymères du styrène réticulés et sulfonés, des résines phénol-acide formaldéhyde sulfonique, et, en tant que catalyseur auxiliaire, en quantité de 0,001 à 0,2 mol/mole de cyclohexanone de formule (III), l'acide β-mercaptopropionique ou la 2-mercaptoéthylamine.
